(19) Europäisches Patentamt
European Patent Office
Office européen des brevets

(11) Veröffentlichungsnummer : **0 158 594**
**B1**

(12) # EUROPÄISCHE PATENTSCHRIFT

(45) Veröffentlichungstag der Patentschrift : **10.02.88**

(21) Anmeldenummer : **85810120.7**

(22) Anmeldetag : **21.03.85**

(51) Int. Cl.⁴ : **C 07 D239/47**, C 07 D239/56, C 07 D239/46, C 07 D239/60, C 07 D239/48, C 07 D239/52

(54) **Verfahren zur Herstellung von Fluoralkoxyaminopyrimidinen.**

(30) Priorität : **27.03.84 US 593218**

(43) Veröffentlichungstag der Anmeldung : **16.10.85 Patentblatt 85/42**

(45) Bekanntmachung des Hinweises auf die Patenterteilung : **10.02.88 Patentblatt 88/06**

(84) Benannte Vertragsstaaten : **AT BE CH DE FR GB IT LI NL SE**

(56) Entgegenhaltungen :
**EP-A- 0 070 802**
**EP-A- 0 070 804**
**EP-A- 0 073 328**
**EP-A- 0 084 020**
**DD-A- 109 170**
**DE-A- 2 533 710**

(73) Patentinhaber : **CIBA-GEIGY AG**
**Klybeckstrasse 141**
**CH-4002 Basel (CH)**

(72) Erfinder : **Pfluger, Rudolf Walter, Dr.**
**Zollweidenstrasse 15**
**CH-4142 Münchenstein (CH)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 158 594 B1**

Jouve, 18, rue St-Denis, 75001 Paris, France

**Beschreibung**

Die vorliegende Erfindung betrifft ein neues Verfahren zur Herstellung von Fluoralkoxyaminopyrimidinen sowie für das neue Verfahren entwickelte Zwischenprodukte.

Die nach dem erfindungsgemässen neuen Verfahren herstellbaren Fluoralkoxyaminopyrimidine sind wertvolle Zwischenprodukte für herbizide und pflanzenwuchsregulierende Wirkstoffe aus der Klasse der Sulfonylharnstoffe. Solche Wirkstoffe sind mit ihren biologischen Wirkungen beispielsweise in den europäischen Patentanmeldungspublikationen EP-A-70802, EP-A-84020 oder EP-A-94790 beschrieben.

Fluoralkoxyaminopyrimidine, die auch nach dem neuen erfindungsgemässen Verfahren herstellbar sind, sind in der europäischen Patentanmeldungspublikation EP-A-70804 zusammen mit einem Verfahren zu ihrer Herstellung sowie Syntheseverfahren zur Ueberführung in die herbiziden und pflanzenwuchsregulierenden Sulfonylharnstoffe beschrieben.

Das in der EP-A-70804 offenbarte Verfahren zur Herstellung von Fluoralkoxy- und Fluoralkylthioaminopyrimidinen durch Umsetzung von entsprechenden Aminopyrimidinen mit Difluorchlormethan, Difluorbrommethan oder Fluoralkenen in Gegenwart von Basen liefert die gewünschten Fluoralkoxyaminopyrimidine in geringen bis mittleren Ausbeuten. Der Umsatz bei diesem Verfahren ist nich vollständig, sodass die Regeneration des Ausgangsmaterials aus den Reaktionslösungen notwendig ist.

Es besteht somit ein Bedürfnis nach einem Verfahren zur Herstellung der Zwischenprodukte, das die Synthese der gewünschten Verbindungen in hohen Ausbeuten zulässt und gleichzeitig die unwirtschaftliche Rückgewinnung unumgesetzen Ausgangsmaterials vermeidet.

Ueberraschenderweise wurde nun festgestellt, dass das erfindungsgemässe neue Verfahren zur Herstellung der Fluoralkoxyaminopyrimidine die Anforderungen des bestehenden Bedürfnisses weitgehend befriedigt.

Nach dem erfindungsgemässen Verfahren können die Fluoralkoxyaminopyrimidine der Formel I

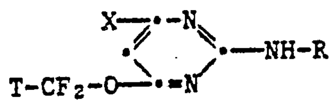

(I)

worin R Wasserstoff, $C_1$-$C_4$-Alkyl, $C_1$-$C_4$-Alkoxy oder Benzyl, X Halogen, $C_1$-$C_4$-Alkyl, $C_1$-$C_4$-Alkoxy, $C_1$-$C_4$-Halogenalkyl, $C_1$-$C_4$-Halogenalkoxy, $C_1$-$C_4$-Alkylamino oder Di-$C_1$-$C_4$-alkylamino, und T Wasserstoff, Chlorfluormethyl, Bromfluormethyl, Difluormethyl oder 1,2,2,2-Tetrafluoräthyl bedeuten, hergestellt werden, indem man ein Thiopyrimidin der Formel II

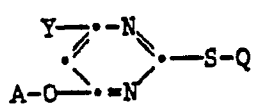

(II)

worin Y die unter Formel I für X gegebene Bedeutung hat oder Hydroxyl bedeutet, Q für $C_1$-$C_4$-Alkyl, Phenyl oder Benzyl und A für Wasserstoff, Natrium, Kalium oder ein Aequivalent von Calcium oder Magnesium stehen, mit Difluorchlormethan, Difluorbrommethan, Tetrafluoräthylen, Perfluorpropylen, Trifluorbromäthylen oder Trifluorchloräthylen in Gegenwart einer Base umsetzt, die erhaltene Verbindung der Formel III

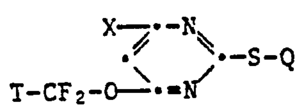

(III)

worin T und X die unter Formel I und Q die unter der Formel II gegebenen Bedeutungen haben, durch Oxidation in eine Verbindung der Formel IV

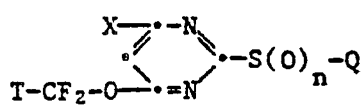

(IV)

worin T und X die unter Formel I und Q die unter der Formel II gegebenen Bedeutungen haben und n für die Zahl eins oder zwei steht, überführt und diese Verbindung mit einem Amin der Formel V

H—NH—R (V),

worin R die unter Formel I gegebene Bedeutung hat, behandelt.

In den Definitionen der Symbole unter den Formeln I, II, III, IV und V umfassen die generischen Begriffe Alkyl, Alkoxy, Halogen oder zusammenfassende Begriffe wie Halogenalkyl, Halogenalkoxy, Alkylamino oder Dialkylamino beispielsweise folgende Bedeutungen : Alkyl steht für Methyl, Aethyl, n-Propyl, i-Propyl oder die vier isomeren Butyl ; Alkoxy steht für Methoxy, Aethoxy, n-Propyloxy, i-Propyloxy oder die vier isomeren Butyloxy ; Halogen bedeutet Fluor, Chlor, Brom oder Jod. Bevorzugte Halogenatome sind Fluor oder Chlor. Die Halogenalkyl-, Halogenalkoxy-, Alkylamino- und Dialkylaminoreste sind sinngemäss zusammengesetzt. Bevorzugte Reste aus diesen Gruppen sind : Fluormethyl, 2,2,2-Trifluoräthoxy, Difluormethoxy, Fluormethoxy, Trifluormethyl, Trifluormethoxy, Perfluoräthyl, 1,1,2,2-Tetrafluoräthoxy, Methylamino, Aethylamino, Dimethylamino oder Diäthylamino. Die Alkylketten in den genannten Resten enthalten vorzugsweise jeweils ein oder zwei Kohlenstoffatome.

Die einzelnen Reaktionsschritte des erfindungsgemässen Verfahrens werden alle mit Vorteil in einem inerten Lösungsmittel oder inerten Lösungsmittelgemisch ausgeführt. Dabei sind auch Lösungsmittelkombinationen eingeschlossen, die aus Lösungsmitteln bestehen, welche untereinander keine homogene Phase bilden. Solche Mischungen sind allgemein als « flüssige Zweiphasensysteme » bekannt.

Der erste Schritt des erfindungsgemässen Verfahrens ist die Fluoralkylierung der freien Hydroxyl-Funktionen des Thiopyrimidines der Formel II. Anstelle der freien Hydroxylverbindungen der Formel II können für diesen Reaktionsschritt auch deren Natrium-, Kalium-, Magnesium- oder Calciumsalze eingesetzt werden. Dieser Reaktionsschritt wird prinzipiell analog zu bekannten Verätherungsreaktionen geführt. Ueberraschenderweise führt die Reaktion der Verbindung der Formel II in hoher Ausbeute zum Zwischenprodukt der Formel III. Als Vorteil hat sich bei diesem Reaktionsschritt die Verwendung eines inerten polaren Lösungsmittels oder Lösungsmittelgemisches erwiesen. Geeignete Lösungsmittel sind : Aether wie Diäthyläther, Aethylenglykoldimethyläther, Diäthylenglykoldimethyläther, Diisopropyläther, Dioxan oder Tetrahydrofuran ; Alkohole wie Methanol, Aethanol, Propanol oder i-Propanol ; Ketone wie Aceton, 2-Butanon oder Cyclohexanon oder Wasser, Dimethylformamid, Acetonitril oder Dimethylsulfoxid. Bevorzugte Lösungsmittel sind Tetrahydrofuran, Dioxan, Methanol, Aethanol, i-Propanol, Aceton, 2-Butanon, Wasser, Dimethylformamid, Acetonitril oder Dimethylsulfoxid. Geeignete Basen sind im allgemeinen anorganische Basen aus der Reihe : Hydride wie Natriumhydrid oder Calciumhydrid ; Oxide wie Magnesiumoxid, Calciumoxid oder Natriumoxid ; Hydroxide wie Lithiumhydroxid, Natriumhydroxid oder Kaliumhydroxid ; oder Carbonate wie Natriumcarbonat oder Kaliumcarbonat. Bei Verwendung gasförmiger oder niedrigsiedender Fluoralkylierungsmittel ist es manchmal angebracht, die Reaktion unter erhöhtem Druck zwischen 1 bar und 100 bar, vorzugsweise zwischen 1 bar und 20 bar, durchzuführen. Geeignete Reaktoren für die Ausführung unter erhöhtem Druck sind Autoklaven. Die Reaktionstemperaturen werden je nach der Beschaffenheit der Reaktionspartner der Formeln II und des Fluoralkylierungsmittels zwischen 0 °C und 120 °C, vorzugsweise zwischen 20 °C und 100 °C gehalten. In geeigneten Fällen kann die Base in Form einer wässrigen Lösung zugesetzt werden. Bildet sich dabei keine homogene Phase, sondern eine zweiphasige Mischung, kann es vorteilhaft sein, dem Reaktionsgemisch einen Phasentransfer-Katalysator, wie beispielsweise ein übliches quaternäres Ammoniumsalz oder einen Kronenäther, zuzusetzen.

Werden Ausgangsprodukte der Formel II eingesetzt, die zwei Hydroxylfunktionen oder Salzformen davon enthalten (Y bedeutet Hydroxyl), so erhält man nur dann einheitliche Reaktionsprodukte, wenn man beide Hydroxylgruppen unter Einsatz entsprechender Mengen an Fluoralkylierungsmittel veräthert.

Eine bevorzugte Ausführungsform der ersten Stufe des erfindungsgemässen Verfahrens besteht darin, dass man die Umsetzung der Verbindung der Formel II zur Verbindung der Formel III bei einer Temperatur zwischen 20 °C und 100 °C, unter einem Druck von 1 bar bis 20 bar, in Gegenwart einer Base aus der Reihe Natriumhydrid, Calciumhydrid, Natriumhydroxid, Kaliumhydroxid, Natriumcarbonat oder Kaliumcarbonat und in einem Lösungsmittel aus der Reihe Dioxan, Tetrahydrofuran, Methanol, Aethanol, i-Propanol, Aceton, 2-Butanon, Wasser, Dimethylformamid, Acetonitril oder Dimethylsulfoxid oder in einem Gemisch dieser Lösungsmittel durchführt.

Der zweite Schritt des erfindungsgemässen Verfahrens ist die Oxidation der —S—Q-Gruppe der Verbindung der Formel III in eine —SO—Q- oder —$SO_2$—Q-Gruppe, wodurch man eine Verbindung der Formel IV erhält. Als Oxidationsmittel dienen übliche Reagenzien wie organische Peroxide, organische Persäuren, Hypochlorite, Wasserstoffperoxid, Kaliumpermanganat, Osmiumtetroxid oder Rutheniumtetroxid. Bevorzugte Oxidationsmittel sind 3-Chlorperbenzoesäure, Perbenzoesäure, Monoperphthalsäure, Peressigsäure, Perameisensäure, Wasserstoffperoxid, Natriumhypochlorit oder Kaliumpermanganat.

Mit Vorteil führt man die Oxidation (III→IV) in einem inerten Lösungsmittel durch. Für die Oxidation bewährte Lösungsmittel sind beispielsweise Methylenchlorid, Chloroform, Tetrachlorkohlenstoff, Tetrachloräthan, Trichloräthan, Chlorbenzol, Dichlorbenzol, Trichlorbenzol, Cyclopentan, Cyclohexan, Essigsäure, Ameisensäure oder Wasser. Bewährt ist auch die Reaktionsführung in Gemischen dieser Lösungsmittel. Im Falle von zweiphasigen flüssigen Lösungsmittelsystemen kann der Zusatz eines üblichen Phasentransferkatalysators, wie zum Beispiel eines quaternären Ammoniumsalzes oder eines Kronenäthers, angezeigt sein. Vorzugsweise wählt man für die Durchführung des Oxidationsschrittes eine Temperatur zwischen — 20 °C und + 120 °C, insbesondere zwischen — 20 °C und + 25 °C.

Eine bevorzugte Ausführungsform der zweiten Stufe des erfindungsgemässen Verfahrens besteht

darin, dass man die Oxidation einer Verbindung der Formel III zu einer Verbindung der Formel IV bei einer Temperatur zwischen — 20 °C und + 25 °C in einem Lösungsmittel aus der Reihe Methylenchlorid, Chloroform, Tetrachlorkohlenstoff, Tetrachloräthan, Trichloräthan, Chlorbenzol, Dichlorbenzol, Trichlorbenzol, Cyclopentan, Cyclohexan, Essigsäure, Ameisensäure oder Wasser oder in einem Gemisch dieser Lösungsmittel unter Verwendung eines Oxidationsmittels aus der Gruppe 3-Chlorperbenzoesäure, Perbenzoesäure, Monoperphthalsäure, Peressigsäure, Perameisensäure, Wasserstoffperoxid, Natriumhypochlorit oder Kaliumpermanganat durchführt.

Die dritte Stufe des erfindungsgemässen Verfahrens ist der Austausch der oxidierten Seitenkette in der 2-Stellung des Pyrimidinringes gegen eine Aminogruppe durch die Behandlung der Verbindung der Formel IV mit einem Amin der Formel V. Auch auf dieser Stufe erweist sich die Reaktionsführung in einem inerten Lösungsmittel oder in einem inerten Lösungsmittelgemisch als vorteilhaft. Geeignete Lösungsmittel sind hierfür Wasser, Methylenchlorid, Chloroform, Tetrachlorkohlenstoff, Tetrachloräthan, Trichloräthan, Chlorbenzol, Diäthyläther, Dioxan, Tetrahydrofuran, Methanol, Aethanol, i-Propanol, Diisopropyläther, Cyclohexan, Dichlorbenzol, Toluol, Xylol, Dimethylformamid, Acetonitril, Dimethylsulfoxid, Aceton, 2-Butanon oder Cyclohexanon. Die Reaktionstemperaturen liegen im allgemeinen zwischen 20 °C und 100 °C. Je nach der Beschaffenheit der Reaktionspartner der Formeln IV und V kann die Anwendung eines erhöhten Druckes von 1 bar bis 100 bar, vorzugsweise von 1 bar bis 20 bar, von Vorteil sein. In diesem Fall kann als Reaktor beispielsweise ein Autoklav eingesetzt werden.

Eine bevorzugte Ausführungsform der dritten Stufe des erfindungsgemässen Verfahrens besteht darin, dass man die Umsetzung einer Verbindung der Formel IV mit einem Amin der Formel V zum Fluoralkoxyaminopyrimidin der Formel I bei einer Temperatur zwischen 20 °C und 100 °C unter einem Druck zwischen 1 bar und 20 bar in einem Lösungsmittel aus der Reihe Wasser, Methylenchlorid, Chloroform, Tetrachlorkohlenstoff, Trichloräthan, Tetrachloräthan, Chlorbenzol, Diäthyläther, Dioxan, Tetrahydrofuran, Methanol, Aethanol, i-Propanol, Diisopropyläther, Dichlorbenzol, Toluol, Xylol, Dimethylformamid, Cyclohexan, Acetonitril, Dimethylsulfoxid, Aceton, 2-Butanon oder Cyclohexanon oder einem Gemisch dieser Lösungsmittel durchführt.

In einer bevorzugten Form des erfindungsgemässen Verfahrens zur Herstellung der Fluoralkoxyaminopyrimidine der Formel I wird das Verfahren so ausgeführt, dass man die Umsetzung der Verbindung der Formel II zur Verbindung der Formel III bei einer Temperatur zwischen 20 °C und 100 °C, unter einem Druck von 1 bar bis 20 bar, in Gegenwart einer Base aus der Reihe Natriumhydrid, Calciumhydrid, Natriumhydroxid, Kaliumhydroxid, Natriumcarbonat oder Kaliumcarbonat und in einem Lösungsmittel aus der Reihe Dioxan, Tetrahydrofuran, Methanol, Aethanol, i-Propanol, Aceton, 2-Butanon, Wasser, Dimethylformamid, Acetonitril oder Dimethylsulfoxid oder einem Gemisch dieser Lösungsmittel durchführt, und dass man die Oxidation der Verbindung der Formel III zu der Verbindung der Formel IV bei einer Temperatur zwischen — 20 °C und + 25 °C in einem Lösungmittel aus der Reihe Methylenchlorid, Chloroform, Tetrachlorkohlenstoff, Tetrachloräthan, Trichloräthan, Chlorbenzol, Dichlorbenzol, Trichlorbenzol, Cyclopentan, Cyclohexan, Essigsäure, Ameisensäure oder Wasser oder einem Gemisch dieser Lösungsmittel unter Verwendung eines Oxidationsmittels aus der Gruppe 3-Chlorperbenzoesäure, Perbenzoesäure, Monoperphthalsäure, Peressigsäure, Perameisensäure, Wasserstoffperoxid, Natriumhypochlorit oder Kaliumpermanganat durchführt und dass man die Umsetzung der Verbindung der Formel IV mit dem Amin der Formel V zum Fluoralkoxyaminpyrimidin der Formel I bei einer Temperatur zwischen 20 °C und 100 °C unter einem Druck zwischen 1 bar und 20 bar in einem Lösungsmittel aus der Reihe Wasser, Methylenchlorid, Chloroform, Tetrachlorkohlenstoff, Trichloräthan, Tetrachloräthan, Chlorbenzol, Diäthyläther, Dioxan, Tetrahydrofuran, Methanol, Aethanol, i-Propanol, Diisopropyläther, Dichlorbenzol, Toluol, Xylol, Dimethylformamid, Cyclohexan, Acetonitril, Dimethylsulfoxid, Aceton, 2-Butanon oder Cyclohexanon oder einem Gemisch dieser Lösungsmittel durchführt.

Gewünschtenfalls können in einer Variante dieses Verfahrens die Umsetzung der Verbindung der Formel II zur Verbindung der Formel III und die Umsetzung der Verbindung der Formel III zur Verbindung der Formel IV unabhängig voneinander in einem flüssigen Zweiphasensystem in Gegenwart eines Phasentransferkatalysators durchgeführt werden. Beispiele für üblicherweise verwendete Phasentransferkatalysatoren sind insbesondere quaternäre Ammoniumsalze, Ammoniumhydroxide, Phosphoniumsalze oder Kronenäther. Als Ammoniumsalze oder -hydroxide kommen vorzugsweise solche aus der Gruppe Benzyltrialkylammonium- oder Tetraalkylammoniumhydroxid, -bisulfat oder -halogenid in Betracht, in denen die Alkylreste zweckmässigerweise 1 bis 4 Kohlenstoffatome enthalten, wie beispielsweise Benzyltriäthylammoniumchlorid, Tetra-n-butylammoniumhydroxid, Benzyltrimethylammoniumchlorid. Besonders geeignet ist ein Tetraalkylammoniumhalogenid, insbesondere Tetra-n-butylammoniumbromid. Beispiele für Phosphoniumsalze sind Tributylhexadecylphosphonium-bromid, Aethyltriphenylphosphonium-bromid, Tetraphenylphosphonium-chlorid, Benzyltriphenylphosphonium-jodid, Triphenyl-n-propylphosphonium-bromid und Tetrabutylphosphoniumchlorid. Als Phasentransferkatalysatoren können im allgemeinen auch folgende Kronenäther eingesetzt werden : 12-Crown-4, 15-Crown-5, 18-Crown-6, Dibenzo-18-crown-6, Dicyclohexano-18-crown-6 oder Dicyclohexano-24-crown-8.

Vorzugsweise wird das erfindungsgemässe Verfahren angewendet, um Verbindungen der Untergruppe Ia

(Siehe Formel Seite 5 f.)

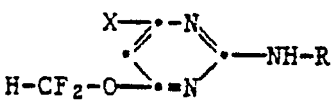

(Ia)

worin R Wasserstoff oder $C_1$-$C_2$-Alkyl und X $C_1$-$C_2$-Alkyl, $C_1$-$C_2$-Alkoxy oder $C_1$-$C_2$-Halogenalkoxy bedeuten, herzustellen. Ganz besonders geeignet ist es jedoch zur Herstellung von 2-Amino-4-difluormethoxy-6-methylpyrimidin.

Als spezifisch bevorzugte Ausführungsform ist das Verfahren anzusehen, welches dadurch gekennzeichnet ist, dass man ein Thiopyrimidin der Formel II in Dioxan in Gegenwart von wässriger Natronlauge bei einer Temperatur zwischen 20 °C und 100 °C mit Difluorchlormethan, Difluorbrommethan, Tetrafluoräthylen, Perfluorpropylen, Trifluorbromäthylen oder Trifluorchloräthylen umsetzt, die erhaltene Verbindung der Formel III in Methylenchlorid mit m-Chlorperbenzoesäure oder in einem Eisessig/Methylenchlorid-Gemisch mit Kaliumpermanganat bei einer Temperatur zwischen —20 °C und +25 °C oder in Eisessig mit wässriger Wasserstoffperoxidlösung bei einer Temperatur zwischen 70 °C und 90 °C oder in Methylenchlorid mit wässriger Natriumhypochlorit-Lösung durch Oxidation in eine Verbindung der Formel IV überführt und diese Verbindung bei einer Temperatur zwischen 20 °C und 100 °C unter einem Druck zwischen 1 bar und 20 bar in einem Methylenchlorid/Wasser-Gemisch mit einem Amin der Formel V behandelt.

Die Ausgangsverbindungen der Formel II sind aus der Literatur bekannt, oder sie können analog zu bekannten Verfahren hergestellt werden. Die Zwischenprodukte der Formel III und der Formel IV sind neu und wurden speziell zur Durchführung des erfindungsgemässen Verfahrens entwickelt. Die Verbindungen der Formel III und IV bilden daher einen Bestandteil der vorliegenden Erfindung. Die Verbindungen der Formel V sind im Handel erhältlich. Angaben über die Herstellung und Eigenschaften der Verbindungen der Formel II findet sich in folgender Literaturstelle : Chem. Listy 52, 357 (1958).

Die Erzeugung der Fluoralkoxyaminopyrimidine der Formel I lässt sich durch die Anwendung des erfindungsgemässen neuen Herstellungsverfahrens in einer speziell an die Erfordernisse der Grosstechnologie angepassten Weise durchführen. Insbesondere wird die Ausbeute an Produkt der Formel I gegenüber dem aus EP-A-70804 bekannten Verfahren erheblich verbessert. Gleichzeitig steigt die Ausbeute an Produkt pro eingesetzte Volumeneinheit. Die Fluoralkylierungsreaktion kann bis zu einem vollständigen Umsatz geführt werden, wodurch der erhebliche verfahrenstechnische Aufwand der Trennung und Regeneration des unverbrauchten Ausgangsmaterials und des Reaktionsproduktes aus der Reaktionslösung erheblich vereinfacht wird.

Die folgenden Beispiele dienen der näheren Illustration der vorliegenden Erfindung. Die Beispiele 2 bis 4, die nur einzelne Reaktionsschritte beschreiben, sind als Varianten der betreffenden Schritte in einer vollständigen Reaktionsfolge zu verstehen.

## Beispiel 1

2-Amino-4-difluormethoxy-6-methylpyrimidin

a) 2-Methylthio-4-difluormethoxy-6-methylpyrimidin. 218,7 g (1,4 Mol) 2-Methylthio-4-hydroxy-6-methylpyrimidin werden in 1,12 Liter Dioxan suspendiert und mit 1 500 g (11,2 Mol) 30 %iger wässriger Natronlauge versetzt. Die Suspension wird auf 80 °C erhitzt. Innerhalb von 3 Stunden leitet man in die heisse Suspension 242 g (2,8 Mol) Difluorchlormethan ein. Die erhaltene blassgelbe Suspension wird auf 20 °C abgekühlt und filtriert. Der Rückstand wird mit Dioxan gewaschen und getrocknet. Man erhält so 210 g 2-Methylthio-4-difluormethoxy-6-methylpyrimidin. Das Filtrat wird mit Methylenchlorid extrahiert, die organische Phase mit Wasser gewaschen und eingedampft. Als kristallinen Rückstand erhält man so nochmals 79 g des gewünschten Produktes. Auf diese Weise erhält man insgesamt 289 g 2-Methylthio-4-difluormethoxy-6-methylpyrimidin (100 % d. Th.) Smp. 40-42 °C.

b) 2-Methylsulfonyl-4-difluormethoxy-6-methylpyrimidin. Zu einer Lösung von 30,9 g (0,15 Mol) 2-Methylthio-4-difluormethoxy-6-methylpyrimidin, 0,75 g 18-Crown-6-äther und 30 ml Eisessig in 1 400 ml Methylenchlorid lässt man eine Lösung von 70,0 g (0,44 Mol) Kaliumpermanganat in 700 ml Wasser zutropfen. Diese Mischung rührt man bei einer Temperatur zwischen 20 °C und 25 °C für 3 Stunden und setzt dann Natriumhydrogensulfitlösung zu, bis das ausgeschiedene Manganoxid ($MnO_2$) gelöst ist. Die organische Phase des Reaktionsgemisches wird abgetrennt, mit Wasser gewaschen, über Natriumsulfat getrocknet und eingedampft. Man erhält so als farblosen Rückstand 33,8 g (95 % d. Th.) 2-Methylsulfonyl-4-difluormethoxy-6-methylpyrimidin, Smp. 88-91 °C.

c) 4,0 g (0,017 Mol) 2-Methylsulfonyl-4-difluormethoxy-6-methylpyrimidin werden in 60 ml Chloroform suspendiert. Diese Suspension wird mit gasförmigem Ammoniak gesättigt und für 3 Stunden bei einer Temperatur zwischen 20 °C und 25 °C gerührt. Anschliessend wird das Reaktionsgemisch mit 50 ml Methylenchlorid und Wasser verdünnt. Die organische Phase wird abgetrennt, und mit 1N Salzsäure extrahiert. Die wässrige Phase wird mit 1N Natronlauge neutralisiert und das ausgefallene Produkt wird abgetrennt, mit Wasser gewaschen und getrocknet. Als kristallinen Rückstand erhält man 2,5 g (85 % d. Th.) 2-Amino-4-difluormethoxy-6-methylpyrimidin, Smp. 137-139 °C.

## Beispiel 2

2-Methylsulfonyl-4-difluormethoxy-6-methylpyrimidin

a) Eine Suspension von 101,5 g (0,5 Mol) 3-Chlorperbenzoesäure in 300 ml Methylenchlorid wird auf eine Temperatur zwischen 0 °C und + 5 °C gekühlt und tropfenweise mit einer Lösung von 2-Methylthio-4-difluormethoxy-6-methylpyrimidin in 50 ml Methylenchlorid versetzt. Nachdem das Reaktionsgemisch für eine weitere Stunde bei gleicher Temperatur gerührt worden ist, werden 100 ml Methylenchlorid zugesetzt, das Gemisch mit Natriumhydrogencarbonatlösung und Wasser gewaschen, über Natriumsulfat getrocknet und eingedampft. Durch Umkristallisieren des Rückstandes aus Methylenchlorid/Petroläther-Gemisch erhält man 45 g (80 % d. Th.) 2-Methylsulfonyl-4-difluormethoxy-6-methylpyrimidin, Smp. 88-91 °C.

b) Eine Lösung von 10,3 g (0,05 Mol) 2-Methylthio-4-difluormethoxy-6-methylpyrimidin in 30 ml Methylenchlorid wird bei 40 °C tropfenweise mit 133,8 g (0,18 Mol) 10 %-iger Natriumhypochlorit-Lösung versetzt. Dabei wird der pH-Wert des Gemisches durch Zusatz von Schwefelsäure bei pH 6 bis pH 8 gehalten. Nach einer Reaktionszeit von 1 Stunde wird die organische Phase abgetrennt und die wässrige Phase mit Methylenchlorid extrahiert. Die vereinigten organischen Phasen werden mit Wasser gewaschen, mit Natriumsulfat getrocknet und eingeengt. Als Rohausbeute erhält man 10,6 g kristallines 2-Methylsulfonyl-4-difluormethoxy-6-methylpyrimidin, Smp. 88-91 °C.

## Beispiel 3

2-Methylsulfinyl-4-difluormethoxy-6-methylpyrimidin

Eine Suspension von 20,6 g (0,1 Mol) 3-Chlorperbenzoesäure in 100 ml Methylenchlorid wird auf eine Temperatur zwischen — 15 °C und — 20 °C abgekühlt und tropfenweise mit einer Lösung von 20,6 g (0,1 Mol) 2-Methylthio-4-difluormethoxy-6-methylpyrimidin in 50 ml Methylenchlorid versetzt. Nachdem das Reaktionsgemisch für 1 Stunde bei der gleichen Temperatur gerührt worden ist, wird es mit Methylenchlorid verdünnt, mit Natriumhydrogencarbonatlösung und Wasser gewaschen, über Natriumsulfat getrocknet und eingedampft. Durch Kristallisation des Rückstandes aus einem Methylenchlorid/Petroläther-Gemisch erhält man 21,6 g (97 % d. Th.) 2-Methylsulfinyl-4-difluormethoxy-6-methylpyrimidin, Smp. 105-107 °C.

## Beispiel 4

2-Methylamino-4-difluormethoxy-6-methylpyrimidin

2,2 g (0,009 Mol) 2-Methylsulfonyl-4-difluormethoxy-6-methylpyrimidin werden bei einer Temperatur zwischen 20 °C und 25 °C mit 30 ml 40 %iger wässriger Methylaminlösung versetzt. Das Gemisch erwärmt sich bis auf eine Temperatur von 60 °C. Das Reaktionsgemisch wird für eine halbe Stunde bei der gleichen Temperatur gerührt, dann abgekühlt und mit 50 ml Methylenchlorid versetzt. Die organische Phase wird abgetrennt, über Natriumsulfat getrocknet und eingedampft. Durch Kristallisation des Rückstandes aus einem Methylenchlorid/Petroläther-Gemisch erhält man 1,4 g (74 % d. Th.) 2-Methylamino-4-difluormethoxy-6-methylpyrimidin, Smp. 129,5-131 °C.

## Beispiel 5

4,6-Bis(difluormethoxy)-2-aminopyrimidin

a) 4,6-Bis(difluormethoxy)-2-methylthiopyrimidin. In ein Gemisch von 130 g 4,6-Dihydroxy-2-methylthiopyrimidin, 750 ml 30 %iger wässriger Natriumhydroxidlösung und 1 000 ml Dioxan leitet man bei einer Temperatur zwischen 75 °C und 80 °C innerhalb von 15 Minuten 160 g Difluorchlormethan ein. Die sich abscheidende organische Phase wird abgetrennt, die wässrige wieder mit 1 000 ml Dioxan versetzt. In dieses Gemisch leitet man erneut 160 g Difluorchlormethan ein. Nach dem Abtrennen der organischen Phase wird dieser Vorgang nochmals wiederholt. Die vereinigten organischen Phasen werden eingedampft und der Rückstand in Eiswasser gegossen. Durch Abtrennen und Trocknen des Niederschlags erhält man 48,2 g (24,8 % d. Th.) 4,6-Bis(difluormethoxy)-2-methylthiopyrimidin, Smp. 46-48 °C.

b) 4,6-Bis(difluormethoxy)-2-methylsulfonylpyrimidin. Eine Lösung von 1,7 g 4,6-Bis(difluormethoxy)-2-methylthiopyrimidin in 20 ml Eisessig wird bei einer Temperatur zwischen 80 °C und 85 °C tropfenweise mit 6,0 ml einer 30 %igen wässrigen Wasserstoffperoxid-Lösung versetzt und für 2 Stunden bei der gleichen Temperatur gerührt. Anschliessend wird das Reaktionsgemisch in Wasser aufgenommen und mit Methylenchlorid extrahiert. Durch Eindampfen der organischen Phase und Umkristallisieren des Rückstandes aus Diäthyläther erhält man 1,8 g (94 % d. Th.) 4,6-Bis(difluormethoxy)-2-methylsulfonylpyrimidin, Smp. 95-96 °C.

c) Einer Lösung von 1,0 g 4,6-Bis(difluormethoxy)-2-methylsulfonylpyrimidin in 20 ml Methylenchlorid setzt man unter kräftigem Rühren 5 ml einer 30 %igen wässrigen Ammoniaklösung zu. Das Reaktionsgemisch erwärmt sich und wird für weitere 2 Stunden gerührt. Durch Eindampfen der Lösung und Kristallisation des Rückstandes erhält man in quantitativer Ausbeute (0,8 g) 4,6-Bis(difluormethoxy)-2-aminopyrimidin, Smp. 67-69 °C.

In analoger Weise erhält man die in den folgenden Tabellen aufgelisteten Zwischen- und Endprodukte. Diese Verbindungen können durch das erfindungsgemässe Verfahren hergestellt werden oder fallen als Zwischenprodukte bei dessen Durchführung an.

Tabelle 1

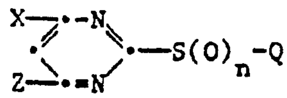

| Verb. Nr. | Q | n | X | Z | phys. Daten |
|---|---|---|---|---|---|
| 1.1 | $CH_3$ | 0 | $CH_3$ | $OCHF_2$ | Smp. 40-42°C |
| 1.2 | $CH_3$ | 1 | $CH_3$ | $OCHF_2$ | Smp. 105-107°C |
| 1.3 | $CH_3$ | 2 | $CH_3$ | $OCHF_2$ | Smp. 88-91°C |
| 1.4 | $C_6H_5$ | 0 | $CH_3$ | $OCHF_2$ | Sdp. 108°C/ 0,04 bar |
| 1.5 | $C_6H_5$ | 2 | $CH_3$ | $OCHF_2$ | Smp. 122-125°C |
| 1.6 | $CH_3$ | 0 | $OCHF_2$ | $OCHF_2$ | Smp. 46-48°C |
| 1.7 | $CH_3$ | 1 | $OCHF_2$ | $OCHF_2$ | Smp. 70-73°C |
| 1.8 | $CH_3$ | 2 | $OCHF_2$ | $OCHF_2$ | Smp. 95-96°C |
| 1.9 | $C_2H_5$ | 0 | $OCHF_2$ | $OCHF_2$ | Smp. 14-16°C Sdp. 85-90°C/ 0,05 bar |
| 1.10 | $C_6H_5-CH_2-$ | 0 | $OCHF_2$ | $OCHF_2$ | Smp. 40-43°C |
| 1.11 | $C_2H_5$ | 2 | $OCHF_2$ | $OCHF_2$ | |
| 1.12 | $C_6H_5-CH_2-$ | 2 | $OCHF_2$ | $OCHF_2$ | |
| 1.13 | $CH_3$ | 0 | $CH_3$ | $OCF_2-CHF_2$ | |
| 1.14 | $CH_3$ | 2 | $CH_3$ | $OCF_2-CHF_2$ | |
| 1.15 | $CH_3$ | 0 | $OCH_3$ | $OCHF_2$ | Smp. 51-52°C |
| 1.16 | $CH_3$ | 1 | $OCH_3$ | $OCHF_2$ | Smp. 75-76°C |
| 1.17 | $CH_3$ | 2 | $OCH_3$ | $OCHF_2$ | Smp. 125-127°C |

Tabelle 2

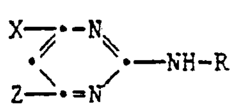

| Verb. Nr. | R | X | Z | phys. Daten |
|---|---|---|---|---|
| 2.1 | H | $CH_3$ | $OCHF_2$ | Smp. 137-139°C |
| 2.2 | $CH_3$ | $CH_3$ | $OCHF_2$ | Smp. 129,5-131°C |

Tabelle 2 (Fortsetzung)

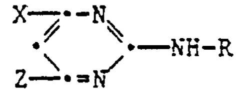

| Verb. Nr. | R | X | Z | phys. Daten |
|---|---|---|---|---|
| 2.3 | H | $OCHF_2$ | $OCHF_2$ | Smp. 67–69°C |
| 2.4 | $C_2H_5$ | $CH_3$ | $OCHF_2$ | Smp. 72–74°C |
| 2.5 | $C_2H_5O$ | $CH_3$ | $OCHF_2$ | Smp. 95–96°C |
| 2.6 | $CH_3$ | $C_2H_5$ | $OCHF_2$ | |
| 2.7 | $CH_3O$ | $CH_3$ | $OCHF_2$ | |
| 2.8 | H | $OCH_3$ | $OCHF_2$ | |
| 2.9 | H | $CH_3$ | $OCF_2CHF_2$ | |
| 2.10 | $C_2H_5$ | $CH_3$ | $OCHF_2$ | |

**Patentansprüche**

1. Verfahren zur Herstellung von Fluoralkoxyaminopyrimidinen der Formel I

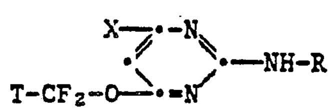

(I)

worin R Wasserstoff, $C_1$-$C_4$-Alkyl, $C_1$-$C_4$-Alkoxy oder Benzyl, X Halogen, $C_1$-$C_4$-Alkyl, $C_1$-$C_4$-Alkoxy, $C_1$-$C_4$-Halogenalkyl, $C_1$-$C_4$-Halogenalkoxy, $C_1$-$C_4$-Alkylamino oder Di-$C_1$-$C_4$-alkylamino und T Wasserstoff, Chlorfluormethyl, Bromfluormethyl, Difluormethyl oder 1,2,2,2-Tetrafluoräthyl bedeuten, dadurch gekennzeichnet, dass man ein Thiopyrimidin der Formel II

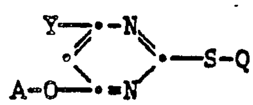

(II)

worin Y die unter Formel I für X gegebene Bedeutung hat oder Hydroxyl bedeutet, Q für $C_1$-$C_4$-Alkyl, Phenyl oder Benzyl und A für Wasserstoff, Natrium, Kalium oder ein Aequivalent von Calcium oder Magnesium stehen, mit Difluorchlormethan, Difluorbrommethan, Tetrafluoräthylen, Perfluorpropylen, Trifluorbromäthylen oder Trifluorchloräthylen in Gegenwart einer Base umsetzt, die erhaltene Verbindung der Formel III

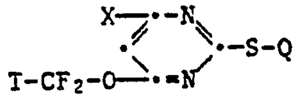

(III)

worin T und X die unter Formel I und Q die unter der Formel II gegebenen Bedeutungen haben, durch Oxidation in eine Verbindung der Formel IV

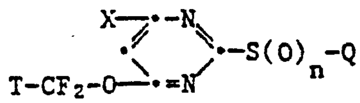

(IV)

worin T und X die unter Formel I und Q die unter der Formel II gegebenen Bedeutungen haben und n für die Zahl eins oder zwei steht, überführt und diese Verbindung mit einem Amin der Formel V

H—NH—R (V),

worin R die unter Formel I gegebene Bedeutung hat, behandelt.

2. Verfahren gemäss Anspruch 1, dadurch gekennzeichnet, dass die einzelnen Reaktionsschritte in Gegenwart inerter Lösungsmittel oder inerter Lösungsmittelgemische durchgeführt werden.

3. Verfahren gemäss Anspruch 1, dadurch gekennzeichnet, dass die Umsetzung der Verbindung der Formel II zur Verbindung der Formel III in Gegenwart eines Lösungsmittels aus der Gruppe Aether, Alkohole, Ketone, Wasser, Dimethylformamid, Acetonitril oder Dimethylsulfoxid oder einem Gemisch davon erfolgt.

4. Verfahren gemäss Anspruch 3, dadurch gekennzeichnet, dass als Lösungsmittel Dioxan, Tetrahydrofuran, Methanol, Aethanol, i-Propanol, Aceton, 2-Butanon, Wasser, Dimethylformamid, Acetonitril oder Dimethylsulfoxid verwendet werden.

5. Verfahren gemäss Anspruch 1, dadurch gekennzeichnet, dass die Umsetzung der Verbindung der Formel II zur Verbindung der Formel III in Gegenwart einer Base aus der Reihe Natriumhydrid, Calciumhydrid, Natriumhydroxid, Kaliumhydroxid, Natriumcarbonat oder Kaliumcarbonat durchgeführt wird.

6. Verfahren gemäss Anspruch 1, dadurch gekennzeichnet, dass die Umsetzung der Verbindung der Formel II zur Verbindung der Formel III unter einem Druck von 1 bar bis 100 bar, vorzugsweise 1 bar bis 20 bar, durchgeführt wird.

7. Verfahren gemäss Anspruch 1, dadurch gekennzeichnet, dass die Umsetzung der Verbindung der Formel II zur Verbindung der Formel III in einem flüssigen Zweiphasensystem in Gegenwart eines Phasentransferkatalysators durchgeführt wird.

8. Verfahren gemäss Anspruch 1, dadurch gekennzeichnet, dass die Umsetzung der Verbindung der Formel II zur Verbindung der Formel III bei einer Temperatur zwischen 0 °C und 120 °C durchgeführt wird.

9. Verfahren gemäss Anspruch 8, dadurch gekennzeichnet, dass die Temperatur zwischen 20 °C und 100 °C liegt.

10. Verfahren gemäss Anspruch 1, dadurch gekennzeichnet, dass die Umsetzung der Verbindung der Formel II zur Verbindung der Formel III bei einer Temperatur zwischen 20 °C und 100 °C, unter einem Druck von 1 bar bis 20 bar, in Gegenwart einer Base aus der Reihe Natriumhydrid, Calciumhydrid, Natriumhydroxid, Kaliumhydroxid, Natriumcarbonat oder Kaliumcarbonat und in einem Lösungsmittel aus der Reihe Dioxan, Tetrahydrofuran, Methanol, Aethanol, i-Propanol, Aceton, 2-Butanon, Wasser, Dimethylformamid, Acetonitril oder Dimethylsulfoxid oder in einem Gemisch dieser Lösungsmittel durchgeführt wird.

11. Verfahren gemäss Anspruch 1, dadurch gekennzeichnet, dass zur Ueberführung der Verbindung der Formel III in eine Verbindung der Formel IV ein Oxidationsmittel aus der Reihe organische Peroxide, organische Persäuren, Hypochlorite, Wasserstoffperoxid, Kaliumpermanganat, Osmiumtetroxid oder Rutheniumtetroxid verwendet wird.

12. Verfahren gemäss Anspruch 11, dadurch gekennzeichnet, dass als Oxidationsmittel 3-Chlorperbenzoesäure, Perbenzoesäure, Monoperphthalsäure, Peressigsäure, Perameisensäure, Wasserstoffperoxid, Natriumhypochlorit oder Kaliumpermanganat verwendet wird.

13. Verfahren gemäss Anspruch 1, dadurch gekennzeichnet, dass die Ueberführung der Verbindung der Formel III in eine Verbindung der Formel IV in einem inerten Lösungsmittel oder einem inerten Lösungsmittelgemisch durchgeführt wird.

14. Verfahren gemäss Anspruch 13, dadurch gekennzeichnet, dass ein Lösungsmittel aus der Reihe Methylenchlorid, Chloroform, Tetrachlorkohlenstoff, Tetrachloräthan, Trichloräthan, Chlorbenzol, Dichlorbenzol, Trichlorbenzol, Cyclopentan, Cyclohexan, Essigsäure, Ameisensäure oder Wasser verwendet wird.

15. Verfahren gemäss Anspruch 1, dadurch gekennzeichnet, dass die Umsetzung der Verbindung der Formel III in eine Verbindung der Formel IV bei einer Temperatur zwischen — 20 °C und + 120 °C, vorzugsweise zwischen — 20 °C und + 25 °C, durchgeführt wird.

16. Verfahren gemäss Anspruch 1, dadurch gekennzeichnet, dass die Oxidation einer Verbindung der Formel III zu einer Verbindung der Formel IV in einem flüssigen Zweiphasensystem in Gegenwart eines Phasentransferkatalysators durchgeführt wird.

17. Verfahren gemäss Anspruch 1, dadurch gekennzeichnet, dass die Oxidation einer Verbindung der Formel III zu einer Verbindung der Formel IV bei einer Temperatur zwischen — 20 °C und + 25 °C in einem Lösungsmittel aus der Reihe Methylenchlorid, Chloroform, Tetrachlorkohlenstoff, Tetrachloräthan, Trichloräthan, Chlorbenzol, Dichlorbenzol, Trichlorbenzol, Cyclopentan, Cyclohexan, Essigsäure, Ameisensäure oder Wasser oder in einem Gemisch dieser Lösungsmittel unter Verwendung eines Oxidationsmittels aus der Gruppe 3-Chlorperbenzoesäure, Perbenzoesäure, Monoperphthalsäure, Peressigsäure, Perameisensäure, Wasserstoffperoxid, Natriumhypochlorit oder Kaliumpermanganat durchgeführt wird.

18. Verfahren gemäss Anspruch 1, dadurch gekennzeichnet, dass die Umsetzung einer Verbindung der Formel IV mit einem Amin der Formel V zum Fluoralkoxyaminopyrimidin der Formel I in einem inerten Lösungsmittel oder einem inerten Lösungsmittelgemisch durchgeführt wird.

19. Verfahren gemäss Anspruch 1, dadurch gekennzeichnet, dass die Umsetzung einer Verbindung

der Formel IV mit einem Amin der Formel V bei einer Temperatur zwischen 20 °C und 100 °C durchgeführt wird.

20. Verfahren gemäss Anspruch 1, dadurch gekennzeichnet, dass die Umsetzung einer Verbindung der Formel IV mit einem Amin der Formel V unter einem Druck zwischen 1 bar und 100 bar, vorzugsweise zwischen 1 bar und 20 bar, durchgeführt wird.

21. Verfahren gemäss Anspruch 18, dadurch gekennzeichnet, dass das Lösungsmittel ausgewählt ist aus der Reihe Wasser, Methylenchlorid, Chloroform, Tetrachlorkohlenstoff, Tetrachloräthan, Trichloräthan, Chlorbenzol, Diäthyläther, Dioxan, Tetrahydrofuran, Methanol, Aethanol, i-Propanol, Diisopropyläther, Cyclohexan, Dichlorbenzol, Toluol, Xylol, Dimethylformamid, Acetonitril, Dimethylsulfoxid, Aceton, 2-Butanon oder Cyclohexanon.

22. Verfahren gemäss Anspruch 1, dadurch gekennzeichnet, dass die Umsetzung einer Verbindung der Formel IV mit einem Amin der Formel V zum Fluoralkoxyaminopyrimidin der Formel I bei einer Temperatur zwischen 20 °C und 100 °C unter einem Druck zwischen 1 bar und 20 bar in einem Lösungsmittel aus der Reihe Wasser, Methylenchlorid, Chloroform, Tetrachlorkohlenstoff, Trichloräthan, Tetrachloräthan, Chlorbenzol, Diäthyläther, Dioxan, Tetrahydrofuran, Methanol, Aethanol, i-Propanol, Diisopropyläther, Dichlorbenzol, Toluol, Xylol, Dimethylformamid, Cyclohexan, Acetonitril, Dimethylsulfoxid, Aceton, 2-Butanon oder Cyclohexanon oder einem Gemisch dieser Lösungsmittel durchgeführt wird.

23. Verfahren gemäss Anspruch 1, dadurch gekennzeichnet, dass die Umsetzung der Verbindung der Formel II zur Verbindung der Formel III bei einer Temperatur zwischen 20 °C und 100 °C, unter einem Druck von 1 bar bis 20 bar, in Gegenwart einer Base aus der Reihe Natriumhydrid, Calciumhydrid, Natriumhydroxid, Kaliumhydroxid, Natriumcarbonat oder Kaliumcarbonat und in einem Lösungsmittel aus der Reihe Dioxan, Tetrahydrofuran, Methanol, Aethanol, i-Propanol, Aceton, 2-Butanon, Wasser, Dimethylformamid, Acetonitril oder Dimethylsulfoxid oder einem Gemisch dieser Lösungsmittel durchgeführt wird, und dass die Oxidation der Verbindung der Formel III zu der Verbindung der Formel IV bei einer Temperatur zwischen — 20 °C und — 25 °C in einem Lösungsmittel aus der Reihe Methylenchlorid, Chloroform, Tetrachlorkohlenstoff, Tetrachloräthan, Trichloräthan, Chlorbenzol, Dichlorbenzol, Trichlorbenzol, Cyclopentan, Cyclohexan, Essigsäure, Ameisensäure oder Wasser oder einem Gemisch dieser Lösungsmittel unter Verwendung eines Oxidationsmittels aus der Gruppe 3-Chlorperbenzoesäure, Perbenzoesäure, Monoperphthalsäure, Peressigsäure, Perameisensäure, Wasserstoffperoxid, Natriumhypochlorit oder Kaliumpermanganat durchgeführt wird, und dass die Umsetzung der Verbindung der Formel IV mit dem Amin der Formel V zum Fluoralkoxyaminpyrimidin der Formel I bei einer Temperatur zwischen 20 °C und 100 °C unter einem Druck zwischen 1 bar und 20 bar in einem Lösungsmittel aus der Reihe Wasser, Methylenchlorid, Chloroform, Tetrachlorkohlenstoff, Trichloräthan, Tetrachloräthan, Chlorbenzol, Diäthyläther, Dioxan, Tetrahydrofuran, Methanol, Aethanol, i-Propanol, Diisopropyläther, Dichlorbenzol, Toluol, Xylol, Dimethylformamid, Cyclohexan, Acetonitril, Dimethylsulfoxid, Aceton, 2-Butanon oder Cyclohexanon oder einem Gemisch dieser Lösungsmittel durchgeführt wird.

24. Verfahren gemäss Anspruch 23, dadurch gekennzeichnet, dass die Umsetzung der Verbindung der Formel II zur Verbindung der Formel III und die Umsetzung der Verbindung der Formel III zur Verbindung der Formel IV unabhängig voneinander in einem flüssigen Zweiphasensystem in Gegenwart eines Phasentransferkatalysators durchgeführt wird.

25. Verfahren gemäss Anspruch 1, dadurch gekennzeichnet, dass man eine Verbindung der Formel Ia

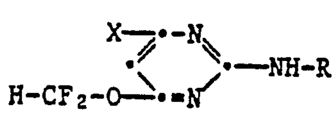

(Ia)

herstellt, worin R Wasserstoff oder $C_1$-$C_2$-Alkyl und X $C_1$-$C_2$-Alkyl, $C_1$-$C_2$-Alkoxy oder $C_1$-$C_2$-Halogenalkoxy bedeuten.

26. Verfahren gemäss Anspruch 1, dadurch gekennzeichnet, dass man 2-Amino-4-difluormethoxy-6-methylpyrimidin herstellt.

27. Verfahren zur Herstellung der Verbindungen der Formel I, dadurch gekennzeichnet, dass man ein Thiopyrimidin der Formel II in Dioxan in Gegenwart von wässriger Natronlauge bei einer Temperatur zwischen 20 °C und 100 °C mit Difluorchlormethan, Difluorbrommethan, Tetrafluoräthylen, Perfluorpropylen, Trifluorbromäthylen oder Trifluorchloräthylen umsetzt, die erhaltene Verbindung der Formel III in Methylenchlorid mit m-Chlorperbenzoesäure oder in einem Eisessig/Methylenchlorid-Gemisch mit Kaliumpermanganat bei einer Temperatur zwischen — 20 °C und + 25 °C oder in Eisessig mit wässriger Wasserstoffperoxidlösung bei einer Temperatur zwischen 70 °C und 90 °C oder in Methylenchlorid mit wässriger Natriumhypochlorit-Lösung durch Oxidation in eine Verbindung der Formel IV überführt und diese Verbindung bei einer Temperatur zwischen 20 °C und 100 °C unter einem Druck zwischen 1 bar und 20 bar in einem Methylenchlorid/Wasser-Gemisch mit einem Amin der Formel V behandelt.

28. Verbindungen der Formel III

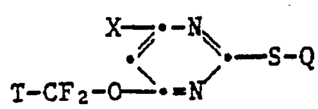

(III)

worin T und X die unter Formel I im Anspruch 1 gegebenen Bedeutungen haben und Q für $C_1$-$C_4$-Alkyl, Phenyl oder Benzyl steht.

29. Verbindungen der Formel IV

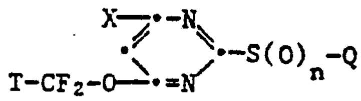

(IV)

worin T und X die unter Formel I im Anspruch 1 gegebenen Bedeutungen haben und Q für $C_1$-$C_4$-Alkyl, Phenyl oder Benzyl und n für die Zahl eins oder zwei stehen.

**Claims**

1. A process for producing fluoroalkoxyaminopyrimidines of the formula I

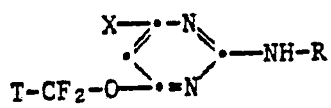

(I)

wherein R is hydrogen, $C_1$-$C_4$-alkyl, $C_1$-$C_4$-alkoxy or benzyl, X is halogen, $C_1$-$C_4$-alkyl, $C_1$-$C_4$-alkoxy, $C_1$-$C_4$-haloalkyl, $C_1$-$C_4$-haloalkoxy, $C_1$-$C_4$-alkylamino or di-$C_1$-$C_4$-alkylamino, and T is hydrogen, chlorofluoromethyl, bromofluoromethyl, difluoromethyl or 1,2,2,2-tetrafluoroethyl, characterised in that a thiopyrimidine of the formula II

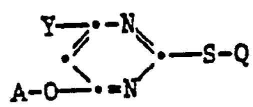

(II)

wherein Y has the meaning given for X under the formula I or is hydroxyl, Q is $C_1$-$C_4$-alkyl, phenyl or benzyl, and A is hydrogen, sodium, potassium or an equivalent of calcium or magnesium, is reacted with difluorochloromethane, difluorobromomethane, tetrafluoroethylene, perfluoropropylene, trifluorobromoethylene or trifluorochloroethylene in the presence of a base ; the resulting compound of the formula III

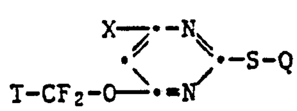

(III)

wherein T and X have the meanings defined under the formula I and Q the meanings defined under the formula II, is converted by oxidation into a compound of the formula IV

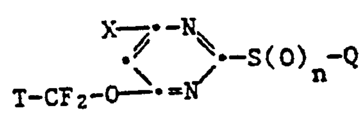

(IV)

wherein T and X have the meanings defined under the formula I and Q the meanings defined under the formula II, and n is the number one or two ; and this compound is treated with an amine of the formula V

H—NH—R (V)

wherein R has the meaning given under the formula I.

2. A process according to claim 1, characterised in that the individual reaction steps are performed in the presence of inert solvents or inert solvent mixtures.

3. A process according to claim 1, characterised in that the reaction of the compound of the formula II to the compound of the formula III is performed in the presence of a solvent selected from the group comprising : ethers, alcohols, ketones, water, dimethylformamide, acetonitrile and dimethyl sulfoxide or a mixture thereof.

4. A process according to claim 3, characterised in that the solvent used is : dioxane, tetrahydrofuran, methanol, ethanol, i-propanol, acetone, 2-butanone, water, dimethylformamide, acetonitrile or dimethyl sulfoxide.

5. A process according to claim 1, characterised in that the reaction of the compound of the formula II to the compound of the formula III is performed in the presence of a base selected from the group comprising : sodium hydride, calcium hydride, sodium hydroxide, potassium hydroxide, sodium carbonate and potassium carbonate.

6. A process according to claim 1, characterised in that the reaction of the compound of the formula II to the compound of the formula III is performed under a pressure of 1 bar to 100 bar, preferably 1 bar to 20 bar.

7. A process according to claim 1, characterised in that the reaction of the compound of the formula II to the compound of the formula III is performed in a liquid two-phase system in the presence of a phase-transfer catalyst.

8. A process according to claim 1, characterised in that the reaction of the compound of the formula II to the compound of the formula III is performed at a temperature of between 0° and 120 °C.

9. A process according to claim 8, characterised in that the temperature is between 20° and 100 °C.

10. A process according to claim 1, characterised in that the reaction of the compound of the formula II to the compound of the formula III is performed at a temperature of between 20° and 100 °C, under a pressure of 1 bar to 20 bar, in the presence of a base selected from the group : sodium hydride, calcium hydride, sodium hydroxide, potassium hydroxide, sodium carbonate and potassium carbonate, and in a solvent selected from the group comprising : dioxane, tetrahydrofuran, methanol, ethanol, i-propanol, acetone, 2-butanone, water, dimethylformamide, acetonitrile and dimethyl sulfoxide, or in a mixture of these solvents.

11. A process according to claim 1, characterised in that there is used for the conversion of the compound of the formula III into a compound of the formula IV an oxidising agent selected from the group : organic peroxides, organic peroxy acids, hypochlorites, hydrogen peroxide, potassium permanganate, osmium tetroxide and ruthenium tetroxide.

12. A process according to claim 11, characterised in that the oxidising agent used is : 3-chloroperoxybenzoic acid, peroxybenzoic acid, monoperoxyphthalic acid, peroxyacetic acid, peroxyformic acid, hydrogen peroxide, sodium hypochlorite or potassium permanganate.

13. A process according to claim 1, characterised in that the conversion of the compound of the formula III into a compound of the formula IV is performed in an inert solvent, or in an inert solvent mixture.

14. A process according to claim 13, characterised in that there is used in a solvent selected from the group comprising : methylene chloride, chloroform, carbon tetrachloride, tetrachloroethane, trichloroethane, chlorobenzene, dichlorobenzene, trichlorobenzene, cyclopentane, cyclohexane, acetic acid, formic acid and water.

15. A process according to claim 1, characterised in that the reaction of the compound of the formula III to a compound of the formula IV is performed at a temperature of between — 20° and + 120 °C, preferably between — 20° and + 25 °C.

16. A process according to claim 1, characterised in that the oxidation of a compound of the formula III to a compound of the formula IV is performed in a liquid two-phase system in the presence of a phase transfer catalyst.

17. A process according to claim 1, characterised in that the oxidation of a compound of the formula III to a compound of the formula IV is performed at a temperature of between — 20° and + 25 °C in a solvent selected from the group comprising : methylene chloride, chloroform, carbon tetrachloride, tetrachloroethane, trichloroethane, chlorobenzene, dichlorobenzene, trichlorobenzene, cyclopentane, cyclohexane, acetic acid, formic acid and water, or in a mixture of these solvents, with the use of an oxidising agent selected from the group comprising : 3-chloroperoxybenzoic acid, peroxybenzoic acid, monoperoxyphthalic acid, peroxyacetic acid, peroxyformic acid, hydrogen peroxide, sodium hypochlorite and potassium permanganate.

18. A process according to claim 1, characterised in that the reaction of a compound of the formula IV with an amine of the formula V to give the fluoroalkoxyaminopyrimidine of the formula I is performed in an inert solvent, or in an inert solvent mixture.

19. A process according to claim 1, characterised in that the reaction of a compound of the formula IV with an amine of the formula V is performed at a temperature of between 20° and 100 °C.

20. A process according to claim 1, characterised in that the reaction of a compound of the formula IV with an amine of the formula V is performed under a pressure of between 1 bar and 100 bar, preferably between 1 bar and 20 bar.

21. A process according to claim 18, characterised in that the solvent is selected from the group comprising : water, methylene chloride, chloroform, carbon tetrachloride, tetrachloroethane, trichloroethane, chlorobenzene, diethyl ether, dioxane, tetrahydrofuran, methanol, ethanol, i-propanol, diisopropyl ether, cyclohexane, dichlorobenzene, toluene, xylene, dimethylformamide, acetonitrile, dimethyl sulfoxide, acetone, 2-butanone and cyclohexanone.

22. A process according to claim 1, characterised in that the reaction of a compound of the formula

IV with an amine of the formula V to give the fluoroalkoxyaminopyrimidine of the formula I is performed at a temperature of between 20° and 100 °C, under a pressure of between 1 bar and 20 bar, in a solvent selected from the group comprising : water, methylene chloride, chloroform, carbon tetrachloride, trichloroethane, tetrachloroethane, chlorobenzene, diethyl ether, dioxane, tetrahydrofuran, methanol, ethanol, i-propanol, diisopropyl ether, dichlorobenzene, toluene, xylene, dimethylformamide, cyclohexane, acetonitrile, dimethyl sulfoxide, acetone, 2-butanone and cyclohexanone, or in a mixture of these solvents.

23. A process according to claim 1, characterised in that the reaction of the compound of the formula II to the compound of the formula III is performed at a temperature of between 20° and 100 °C, under a pressure of 1 bar to 20 bar, in the presence of a base selected from the group comprising : sodium hydride, calcium hydride, sodium hydroxide, potassium hydroxide, sodium carbonate and potassium carbonate, and in a solvent selected from the group comprising : dioxane, tetrahydrofuran, methanol, ethanol, i-propanol, acetone, 2-butanone, water, dimethylformamide, acetonitrile and dimethyl sulfoxide, or in a mixture of these solvents ; and that the oxidation of the compound of the formula III to the compound of the formula IV is performed at a temperature of between — 20° and — 25 °C in a solvent selected from the group comprising : methylene chloride, chloroform, carbon tetrachloride, tetrachloroethane, trichloroethane, chlorobenzene, dichlorobenzene, trichlorobenzene, cyclopentane, cyclohexane, acetic acid, formic acid and water, or in a mixture of these solvents, with the use of an oxidising agent selected from the group comprising : 3-chloroperoxybenzoic acid, peroxybenzoic acid, monoperoxyphthalic acid, peroxyacetic acid, peroxyformic acid, hydrogen peroxide, sodium hypochlorite and potassium permanganate ; and that the reaction of the compound of the formula IV with the amine of the formula V to give the fluoroalkoxyaminopyrimidine of the formula I is performed at a temperature of between 20° and 100 °C, under a pressure of between 1 bar and 20 bar, in a solvent selected from the group comprising : water, methylene chloride, chloroform, carbon tetrachloride, trichloroethane, tetrachloroethane, chlorobenzene, diethyl ether, dioxane, tetrahydrofuran, methanol, ethanol, i-propanol, diisopropyl ether, dichlorobenzene, toluene, xylene, dimethylformamide, cyclohexane, acetonitrile, dimethyl sulfoxide, acetone, 2-butanone and cyclohexanone, or in a mixture of these solvents.

24. A process according to claim 23, characterised in that the reaction of the compound of the formula II to the compound of the formula III and the reaction of the compound of the formula III to the compound of the formula IV are performed independently of one another in a liquid two-phase system in the presence of a phase-transfer catalyst.

25. A process according to claim 1, characterised in that there is produced a compound of the formula Ia

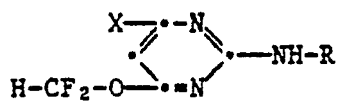

(Ia)

wherein R is hydrogen or $C_1$-$C_2$-alkyl, and X is $C_1$-$C_2$-alkyl, $C_1$-$C_2$-alkoxy or $C_1$-$C_2$-haloalkoxy.

26. A process according to claim 1, characterised in that 2-amino-4-difluoromethoxy-6-methylpyrimidine is produced.

27. A process for producing the compounds of the formula I, characterised in that a thiopyrimidine of the formula II is reacted in dioxane in the presence of an aqueous sodium hydroxide solution, at a temperature of between 20° and 100 °C, with difluorochloromethane, difluorobromomethane, tetrafluoroethylene, perfluoropropylene, trifluorobromoethylene or trifluorochloroethylene, the resulting compound of the formula III is converted in methylene chloride with m-chloroperoxybenzoic acid, or in a glacial acetic acid/methylene chloride mixture with potassium permanganate, at a temperature of between — 20° and + 25 °C, or in glacial acetic acid with aqueous hydrogen peroxide solution at a temperature of between 70° and 90 °C or in methylene chloride with aqueous sodium hypochlorite solution, by oxidation into a compound of the formula IV, and this compound is treated at a temperature of between 20° and 100 °C, under a pressure of between 1 bar and 20 bar, in a methylene chloride/water mixture with an amine of the formula V.

28. Compounds of the formula III

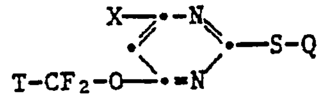

(III)

wherein T and X have the meanings defined under the formula I in claim 1, and Q is $C_1$-$C_4$-alkyl, phenyl or benzyl.

29. Compounds of the formula IV

(See formula page 14)

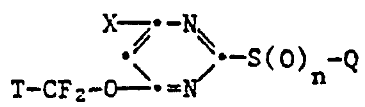

(IV)

wherein T and X have the meanings defined under the formula I in claim 1, and Q is $C_1$-$C_4$-alkyl, phenyl or benzyl, and n is the number one or two.

## Revendications

1. Procédé de préparation de fluoroalcoxyaminopyrimidine de formule I

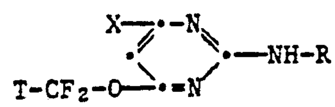

(I)

dans laquelle R représente un hydrogène, un alcoyle en $C_1$ à $C_4$, un alcoxy en $C_1$ à $C_4$ ou un benzyle, X représente un halogène, un alcoyle en $C_1$ à $C_4$, un alcoxy en $C_1$ à $C_4$, un halogènealcoyle en $C_1$ à $C_4$, un halogènealcoxy en $C_1$ à $C_4$, un alcoylamino en $C_1$ à $C_4$ ou un di-alcoylamino en $C_1$ à $C_4$ et T représente un hydrogène, chlorofluorométhyle, bromofluorométhyle, difluorométhyle ou 1,2,2,2-tétrafluoroéthyle, caractérisé en ce qu'on fait réagir une thiopyrimidine de formule II

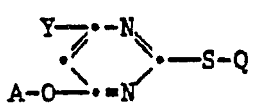

(II)

dans laquelle Y a la signification donnée sous la formule I pour X ou représente un hydroxy, Q représente un alcoyle en $C_1$ à $C_4$, un phényle ou un benzyle et A représente un hydrogène, sodium, potassium ou un équivalent de calcium ou de magnésium, avec du difluorochlorométhane, difluorobromométhane, tétrafluoroéthylène, perfluoropropylène, trifluorobrométhylène ou trifluorochloroéthylène en présence d'une base, en ce qu'on transforme le composé obtenu de formule III

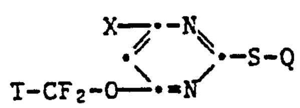

(III)

dans laquelle T et X ont les significations données sous la formule I et Q la signification donnée sous la formule II, par oxydation en un composé de formule IV

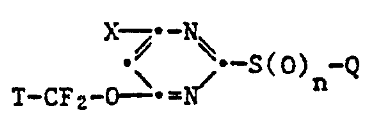

(IV)

dans laquelle T et X ont la signification donnée sous la formule I et Q la signification donnée sous la formule II et n représente le nombre un ou deux, et on traite ce composé avec une amine de formule V

$$H—NH—R \quad (V),$$

dans laquelle R a la signification donnée sous la formule I.

2. Procédé selon la revendication 1, caractérisé en ce que les diverses étapes de la réaction sont conduites en présence de solvants inertes ou de mélanges de solvants inertes.

3. Procédé selon la revendication 1, caractérisé en ce que la réaction du composé de formule II pour donner un composé de formule III s'effectue en présence d'un solvant du groupe éthers, alcools, cétones, eau, diméthylformamide, acétonitrile ou diméthylsulfoxyde ou d'un de leurs mélanges.

4. Procédé selon la revendication 3, caractérisé en ce qu'on utilise comme solvants le dioxanne, tétrahydrofuranne, méthanol, éthanol, i-propanol, acétone, 2-butanone, eau, diméthylformamide, acétonitrile ou diméthylsulfoxyde.

5. Procédé selon la revendication 1, caractérisé en ce que la réaction du composé de formule II pour donner un composé de formule III est conduite en présence d'une base de la série hydrure de sodium, hydrure de calcium, hydroxyde de sodium, hydroxyde de potassium, carbonate de sodium ou carbonate de potassium.

6. Procédé selon la revendication 1, caractérisé en ce que la réaction du composé de formule II pour donner un composé de formule III est conduite sous une pression allant de 1 bar à 100 bars, de préférence de 1 bar à 20 bars.

7. Procédé selon la revendication 1, caractérisé en ce que la réaction du composé de formule II pour donner un composé de formule III est conduite dans un système à deux phases liquides en présence d'un catalyseur de transfert de phase.

8. Procédé selon la revendication 1, caractérisé en ce que la réaction du composé de formule II pour donner un composé de formule III est conduite à une température comprise entre 0 °C et 120 °C.

9. Procédé selon la revendication 8, caractérisé en ce que la température est comprise entre 20 °C et 100 °C.

10. Procédé selon la revendication 1, caractérisé en ce que la réaction du composé de formule II pour donner un composé de formule III est conduite à une température comprise entre 20 °C et 100 °C, sous une pression de 1 bar à 20 bars, en présence d'une base de la série hydrure de sodium, hydrure de calcium, hydroxyde de sodium, hydroxyde de potassium, carbonate de sodium ou carbonate de potassium et dans un solvant de la série dioxanne, tétrahydrofuranne, méthanol, éthanol, i-propanol, acétone, 2-butanone, eau, diméthylformamide, acétonitrile ou diméthylsulfoxyde ou dans un mélange de ces solvants.

11. Procédé selon la revendication 1, caractérisé en ce que pour transformer le composé de formule III en un composé de formule IV on utilise un oxydant de la série peroxydes organiques, peracides organiques, hypochlorytes, peroxydes d'hydrogène, permanganate de potassium, tétroxydes d'osmium ou tétroxydes de ruthénium.

12. Procédé selon la revendication 11, caractérisé en ce qu'on utilise comme oxydants l'acide 3-chloroperbenzoïque, l'acide perbenzoïque, l'acide monoperphtalique, l'acide peracétique, l'acide performique, le peroxyde d'hydrogène, l'hypochlorite de sodium ou le permanganate de potassium.

13. Procédé selon la revendication 1, caractérisé en ce qu'on conduit la transformation du composé de formule III en un composé de formule IV dans un solvant inerte ou dans un mélange de solvants inertes.

14. Procédé selon la revendication 13, caractérisé en ce qu'on utilise un solvant de la série chlorure de méthylène, chloroforme, tétrachlorure de carbone, tétrachloroéthane, trichloroéthane, chlorobenzène, dichlorobenzène, trichlorobenzène, cyclopentane, cyclohexane, acide acétique, acide formique ou eau.

15. Procédé selon la revendication 1, caractérisé en ce qu'on conduit la transformation du composé de formule III en un composé de formule IV à une température comprise entre — 20 °C et + 120 °C, de préférence entre — 20 °C et + 25 °C.

16. Procédé selon la revendication 1, caractérisé en ce qu'on conduit l'oxydation d'un composé de formule III en un composé de formule IV dans un système à deux phases liquides en présence d'un catalyseur de transfert de phase.

17. Procédé selon la revendication 1, caractérisé en ce qu'on conduit l'oxydation d'un composé de formule III en un composé de formule IV à une température comprise entre — 20 °C et + 25 °C dans un solvant de la série chlorure de méthylène, chloroforme, tétrachlorure de carbone, tétrachloroéthane, trichloroéthane, chlorobenzène, dichlorobenzène, trichlorobenzène, cyclopentane, cyclohexane, acide acétique, acide formique ou eau ou dans un mélange de ces solvants en utilisant un oxydant du groupe acide 3-chloroperbenzoïque, acide perbenzoïque, acide monoperphtalique, acide peracétique, acide performique, peroxyde d'hydrogène, hypochlorite de sodium ou permanganate de potassium.

18. Procédé selon la revendication 1, caractérisé en ce qu'on conduit la réaction d'un composé de formule IV avec une amine de formule V pour donner une fluoroalcoxyaminopyrimidine de formule I dans un solvant inerte ou un mélange de solvants inertes.

19. Procédé selon la revendication 1, caractérisé en ce qu'on conduit la réaction d'un composé de formule IV avec une amine de formule V à une température comprise entre 20 °C et 100 °C.

20. Procédé selon la revendication 1, caractérisé en ce qu'on conduit la réaction d'un composé de formule IV avec une amine de formule V sous une pression comprise entre 1 bar et 100 bars, de préférence entre 1 bar et 20 bars.

21. Procédé selon la revendication 18, caractérisé en ce que le solvant est choisi dans la série eau, chlorure de méthylène, chloroforme, tétrachlorure de carbone, tétrachloroéthane, trichloroéthane, chlorobenzène, diéthyléther, dioxanne, tétrahydrofuranne, méthanol, éthanol, i-propanol, diisopropyléther, cyclohexane, dichlorobenzène, toluène, xylène, diméthylformamide, acétonitrile, diméthylsulfoxyde, acétone, 2-butanone ou cyclohexanone.

22. Procédé selon la revendication 1, caractérisé en ce que la réaction d'un composé de formule IV avec une amine de formule V pour donner la fluoroalcoxyaminopyrimidine de formule I est conduite à une température comprise entre 20 °C et 100 °C sous une pression comprise entre 1 bar et 20 bars dans un solvant de la série eau, chlorure de méthylène, chloroforme, tétrachlorure de carbone, trichloroéthane, tétrachloroéthane, chlorobenzène, diéthyléther, dioxanne, tétrahydrofuranne, méthanol, éthanol, i-propanol, diisopropyléther, dichlorobenzène, toluène, xylène, diméthylformamide, cyclohexane, acétonitrile, diméthylsulfoxyde, acétone, 2-butanone ou cyclohexanone ou un mélange de ces solvants.

23. Procédé selon la revendication 1, caractérisé en ce que la transformation du composé de formule II en composé de formule III est conduite à une température comprise entre 20 °C et 100 °C, sous une

pression allant de 1 bar à 20 bars, en présence d'une base de la série hydrure de sodium, hydrure de calcium, hydroxyde de sodium, hydroxyde de potassium, carbonate de sodium ou carbonate de potassium et dans un solvant de la série dioxanne, tétrahydrofuranne, méthanol, éthanol, i-propanol, acétone, 2-butanone, eau, diméthylformamide, acétonitrile ou diméthylsulfoxyde ou dans un mélange de ces solvants, et en ce que l'oxydation du composé de formule III en le composé de formule IV est conduite à une température comprise entre — 20 °C et — 25 °C dans un solvant de la série chlorure de méthylène, chloroforme, tétrachlorure de carbone, tétrachloroéthane, trichloroéthane, chlorobenzène, dichlorobenzène, trichlorobenzène, cyclopentane, cyclohexane, acide acétique, acide formique ou eau ou un mélange de ces solvants en utilisant un oxydant du groupe acide 3-chloroperbenzoïque, acide perbenzoïque, acide monoperphtalique, acide peracétique, acide performique, peroxyde d'hydrogène, hypochlorite de sodium ou permanganate de potassium, et en ce que la réaction du composé de formule IV avec l'amine de formule V pour donner la fluoroalcoxyaminopyrimidine de formule I est conduite à une température comprise entre 20 °C et 100 °C sous une pression comprise entre 1 bar et 20 bars dans un solvant de la série eau, chlorure de méthylène, chloroforme, tétrachlorure de carbone, trichloroéthane, tétrachloroéthane, chlorobenzène, diéthyléther, dioxanne, tétrahydrofuranne, méthanol, éthanol, i-propanol, diisopropyléther, dichlorobenzène, toluène, xylène, diméthylformamide, cyclohexane, acétonitrile, diméthylsulfoxyde, acétone, 2-butanone ou cyclohexanone ou un mélange de ces solvants.

24. Procédé selon la revendication 23, caractérisé en ce que la transformation du composé de formule II en composé de formule III et en ce que la transformation du composé de formule III en composé de formule IV sont conduites indépendamment l'une de l'autre dans un système à deux phases liquides en présence d'un catalyseur de transfert de phase.

25. Procédé selon la revendication 1, caractérisé en ce qu'on prépare un composé de formule Ia

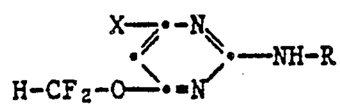

(Ia)

dans laquelle R représente un hydrogène ou un alcoyle en $C_1$ à $C_2$ et X un alcoyle en $C_1$ à $C_2$, alcoxy en $C_1$ à $C_2$ ou halogène alcoxy en $C_1$ à $C_2$.

26. Procédé selon la revendication 1, caractérisé en ce qu'on prépare la 2-amino-4-difluorométhoxy-6-méthylpyrimidine.

27. Procédé de préparation des composés de formule I, caractérisé en ce qu'on fait réagir une thiopyrimidine de formule II dans le dioxanne en présence de lessive de soude aqueuse à une température comprise entre 20 °C et 100 °C avec un difluorochlorométhane, difluorobromométhane, tétrafluoroéthylène, perfluoropropylène, trifluorobrométhylène ou trifluorochloroéthylène, en ce qu'on transforme le composé obtenu de formule III dans le chlore de méthylène avec de l'acide m-chloroperbenzoïque ou dans un mélange acide acétique glacial/chlorure de méthylène avec du permanganate de potassium à une température comprise entre — 20 °C et + 25 °C ou dans l'acide acétique glacial avec une solution aqueuse du peroxyde d'hydrogène à une température comprise entre 70 °C et 90 °C ou dans le chlorure de méthylène avec une solution aqueuse d'hypochlorite de sodium par oxydation en un composé de formule IV et en ce qu'on traite ce composé à une température comprise entre 20 °C et 100 °C sous une pression comprise entre 1 bar et 20 bars dans un mélange chlorure de méthylène/eau avec une amine de formule V.

28. Composés de formule III

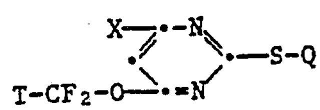

(III)

dans laquelle T et X ont les significations données sous la formule I dans la revendication 1 et Q représente un alcoyle en $C_1$ à $C_4$, phényle ou benzyle.

29. Composés de formule IV

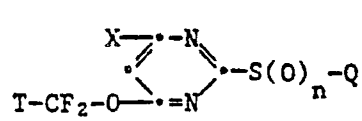

(IV)

dans laquelle T et X ont les significations données sous la formule I dans la revendication 1 et Q représente un alcoyle en $C_1$ à $C_4$, phényle ou benzyle et n représente le nombre un ou deux.